# EUROPEAN PATENT SPECIFICATION

(11) **EP 3 846 884 B1**
(45) Date of publication and mention of the grant of the patent: **31.01.2024**
(21) Application number: 19766307.3
(22) Date of filing: 03.09.2019
(51) Int. Cl.: A61M 15/00

(54) **MEDICAMENT DELIVERY DEVICE AND METHODS**
MEDIKAMENTENABGABEVORRICHTUNG UND VERFAHREN
DISPOSITIF ET PROCÉDÉS D'ADMINISTRATION DE MÉDICAMENT

(30) Priority: 03.09.2018 GB 201814300
(43) Date of publication of application: 14.07.2021
(73) Proprietor: TTP PLC, Royston, Hertfordshire SG8 6EE (GB)
(72) Inventor: COTTENDEN, David, Royston, Hertfordshire SG8 6EE (GB); WANG, Jianye, Royston, Hertfordshire SG8 6EE (GB); YOUNG, Duncan, Royston, Hertfordshire SG8 6EE (GB); CHEUNG, Desmond, Royston, Hertfordshire SG8 6EE (GB); WADDELOW, Simon, Royston, Hertfordshire SG8 6EE (GB); MCDERMENT, Iain, Royston, Hertfordshire SG8 6EE (GB); SELBY, Rob, Royston, Hertfordshire SG8 6EE (GB)
(74) Representative: Gill Jennings & Every LLP
(86) International application number: PCT/GB2019/052450
(87) International publication number: WO 2020/049288

(56) References cited:
- WO-A1-03/061743
- WO-A1-03/061744
- WO-A1-2010/133323

## Description

### Background

The present invention relates to medicament delivery devices, and more specifically devices and methods for the delivery of aerosolised medicament. In particular, the present invention relates to improved multi-unit dose (MUD) dry powder inhalers (DPIs) and mechanisms for releasing the doses of medicament, as defined by the present claims.

An increasing number of inhaled drug therapies involve combinations of two dry powder formulations, which are not stable when mixed. This leaves the option of delivering the formulations using two different inhalers, or creating a device which stores them separately until each medicament dose is delivered. The latter option is much more attractive, since it is far easier for the user, and would be expected to increase the probability of adherence to the prescribed dosing regimen.

A known device that can store such formulations separately until delivery of the medicament doses is described in EP2929905, WO 2003/061743 and WO 2005/014089.

This device contains two medicament carriers (e.g. "blister strips"), each one of which comprises a carrier strip in which a number of compartments (e.g. depressions) have been formed. The compartments are regularly spaced apart along the carrier strip, and the majority of the compartments are each filled with a measured dose of medicament, typically in the form of a dry powder. The two medicament carriers each carry different medicaments, which are mixed immediately prior to inhalation. A sealing layer (e.g. a laminated backing) is secured (e.g. welded) over the carrier strip to seal the compartments and provide a moisture barrier sealing the medicament in the individual compartments (or "blisters"). A peeling mechanism is arranged to peel a portion of the sealing layer away from each carrier strip when the device is primed and present the exposed compartments to an aerosolisation chamber in which the formulation is aerosolised when a user inhales through the device.

The peeling (e.g. "release") mechanism of the known device requires the incoming (sealed) carrier strip, the outgoing (opened) carrier strip, and the outgoing backing (or "sealing layer") to be separately handled, spooled and tensioned. In particular, the incoming strip and the outgoing backing need to have location and tension (respectively) tightly controlled. Since there are two strips, this means that a total of six components (i.e. the incoming and outgoing carrier strips and the outgoing backing of each medicament carrier) have to be controlled, co-ordinated, and - for two of them - tensioned.

This device therefore requires numerous mechanical parts to handle the two blister strips and their separated components. Requiring two of every component inside the medicament delivery device results in a device composed of many parts. As a result, this device is presumed to be both complicated and expensive to produce. In particular, installing the strips at the correct tension would seem to be complicated.

A problem with known peel-based DPI devices is that during release of a medicament dose from its compartment on the blister (or "carrier") strip, the powder can be dropped inside the device (potentially the whole dose), for example if the device is knocked mid-actuation.

Other multi-unit dose (MUD) DPIs do exist; such devices typically either have a similar peeling mechanism as described above or rely on cutting or tearing a thinner backing material.

A problem with cutting and tearing mechanisms, however, is that direction and timing of a cut or tear can be difficult to predict, and such devices therefore often struggle to eliminate chads and shards of backing. To avoid these problems, a cutting blade in the device would have to be sharper than is easy to achieve in polymer blades, and steel blades are more expensive to produce and assemble.

Furthermore, both tearing and cutting have the additional disadvantage that the cut or torn edges of the backing remain in or near the airpath, where they can affect the powder dispersion process in difficult-to-predict ways. These are significant challenges to address.

An improved dry powder inhaler (DPI) device and a simpler release mechanism that addresses these challenges is therefore desirable, and preferably a device that can deliver two mutually unstable (and hence separately stored) formulations in discrete unit doses. Such a simpler device or mechanism may have a substantially smaller part count, which should therefore make it less expensive, and less complex to manufacture.

A device that can deliver a more reliable multi-unit dosage with the opening of the powder-containing compartments being more predictable and repeatable is also desirable.

### Summary of the present invention

The device and mechanisms described herein employ release mechanisms that provide a more predictable and repeatable opening (or peeling) of compartments than known devices. They also provides "*in situ*" opening and simpler strip handling than cutting mechanisms, thereby enabling a low part-count device without critical dependence on difficult-to-control material properties of the backing and welding parameters, or development difficulties around chad formation, as required by known devices.

According to one aspect of the present invention there is provided a device for releasing discrete doses of medicament carried in a plurality of spaced-apart compartments on a medicament carrier, each compartment being arranged in a carrier strip and sealed by a sealing layer, the device comprising a release mechanism arranged to engage between the carrier strip and the sealing layer so as to unseal at least a portion of the compartment and expose the medicament dose contained therein, as defined by the present claims.

As described herein, the terms "unseal", "separate", "remove" and "release" (in relation to the sealing layer) preferably connote to "peel away" at least a portion of the sealing layer from the carrier strip to expose the medicament (dose) contained therein. This is in contrast to applying pressure to (e.g. the underside of) the carrier strip and hence its contents thereby causing the sealing layer to rupture and expose the contents of the compartment, for example.

As well as the advantage of fewer elements to handle, the device combines very naturally with in-built air paths, meaning that the compartment carrying the medicament dose (e.g. dry powder) is not opened until it is in the necessary position to enable the exposed medicament dose to be inhaled, thereby mitigating the risk of powder loss if the device is knocked.

The carrier strip may pass through the release mechanism when engaged. The release mechanism may be arranged to receive the compartment while said at least a portion of compartment is unsealed.

The release mechanism may be arranged to remove a portion of the sealing layer from the compartment while leaving a remaining portion of the sealing layer over the compartment. The release mechanism may be arranged to engage and remove at least a portion of the sealing layer from at least one side of the compartment. Preferably, the release mechanism may be arranged to remove portions of the sealing layer from substantially opposed sides of the compartment such that the remaining portion is located between the opposed sides.

The device may further comprise means for engaging with a portion of the sealing layer such that contact is maintained between at least a portion of the sealing layer and the carrier strip when the release mechanism engages the medicament carrier. This ensures that the remaining portion of the sealing layer, which is not being removed from the compartment, is held in place during the removal process. Preferably, the engaging means engages with a substantially central portion of the sealing layer. This ensures that only the end portions of the compartment are unsealed by the release mechanism.

The means for engaging may be a protuberance arranged to contact the sealing layer, preferably to bias or otherwise urge it against the carrier strip. The protuberance may be part of the release mechanism, or it may be part of a housing that contains the release mechanism, such as an internal wall for example.

The release mechanism may be arranged to separate the sealing layer from the carrier strip such that the sealing layer is completely detached across the full width of the carrier strip over a compartment thereby completely exposing the compartment.

The release mechanism may comprise at least one release member arranged to engage between the compartment and sealing layer whereby to remove said at least a portion of the sealing layer. The at least one release member may be arranged to engage the medicament carrier such that the carrier strip passes beneath the at least one release member and the sealing layer passes over the at least one release member.

The at least one release member may have a lower surface arranged to pass closely over each compartment in the carrier strip as the medicament carrier is advanced through the release mechanism, for example so as to substantially seal the portion of compartment where the sealing layer is removed.

The at least one release member may have a leading edge arranged to engage between the carrier strip and sealing layer so as to separate the sealing layer from the carrier strip as the carrier moves past the release mechanism. The leading edge may have a curved, raised profile along its length, for example raised in a direction out of the plane of the direction (e.g. path) of travel of the medicament carrier.

The at least one release member may have a leading edge configured to conform in part to the shape of the perimeter of a compartment, preferably in the same plane as (e.g. across) the sealing layer. This configuration may allow the release member to remove as much of the sealing layer as possible while avoiding opening the next compartment immediately upstream of the element. In addition, this configuration may allow as much of the sealing layer as possible to be removed from the carrier strip.

The at least one release member may have a trailing edge shaped to conform in part to the shape of the perimeter of a compartment, preferably in the same plane as (e.g. across) the sealing layer. This configuration may avoid covering the compartment previously opened immediately downstream of the element. In addition, this configuration allows as much of the sealing layer as possible to be removed from the carrier strip without overlapping the previous compartment to avoid inhalation of any previously un-inhaled medicament from the previous compartment.

At least one of the leading edge and the trailing edge of the at least one release member may have a generally curved configuration. The at least one release member may be arranged to extend substantially the entire width of the medicament carrier.

The release mechanism may of the invention comprises two release members. The two release members may be arranged in a substantially opposed configuration, such that they are opposed across the medicament carrier.

In some embodiments the two release members may be spaced apart from each other. Thus, a gap may be present between the two release members. In other embodiments, the two release members may be arranged to be coupled together so as to extend substantially the entire width of the medicament carrier. The at least one projecting member may be removable from the release mechanism so as to facilitate engagement with the medicament carrier. The release mechanism may comprise two separable parts that are configured to be coupled together so as to engage with the medicament carrier.

The release mechanism may be arranged to remove at least a portion of the sealing layer covering a compartment and to cover said compartment in place of said removed portion of sealing layer, wherein the release mechanism may be further arranged to provide a fluid conduit configured to pass air into or through the compartment so as to aerosolise the medicament contained therein. The release mechanism preferably covers said exposed compartment so as substantially to seal the compartment in place of the sealing layer such that air can only pass through (e.g. or enter) said compartment via the release mechanism. In other words, due to the presence of the release mechanism (preferably, a release member thereof), an exposed compartment cannot be accessed from directly above because it is covered, by a mouthpiece positioned directly above the compartment, for example. As such, the release mechanism may provide a fluid conduit that passes air through the compartment, wherein the compartment forms part of the fluid conduit. For example, due to the exposed compartment being covered, the aerosolised medicament dose from said compartment travels along at least a portion of the release mechanism before it is free of the medicament carrier, preferably along an underside of the release mechanism that faces the exposed compartment, and preferably across a portion of the carrier strip adjacent the compartment, for example the edge of the carrier strip. The direction of airflow may therefore be across the width of the carrier strip, preferably in a direction transverse to the length of the carrier strip.

According to another aspect of the present invention there is provided a device for releasing discrete doses of medicament carried in a plurality of spaced-apart compartments on a medicament carrier, each compartment being arranged in a carrier strip and sealed by a sealing layer, the device comprising a release mechanism arranged to remove at least a portion of the sealing layer covering a compartment and to cover said compartment in place of said removed portion of sealing layer, wherein the release mechanism is further arranged to provide a fluid conduit configured to pass air into or through the compartment so as to aerosolise the medicament contained therein.

The release mechanism may be fluidly connectable with a mouthpiece for inhalation such that medicament can be dispensed from a compartment that is at least partially exposed during engagement of the release mechanism with the medicament carrier.

As noted above, the release mechanism preferably covers said exposed compartment so as substantially to seal the compartment in place of the sealing layer such that air can only pass through (e.g. or enter) said compartment via the release mechanism. In other words, due to the presence of the release mechanism (preferably, a release member thereof), an exposed compartment cannot be inhaled directly into a mouthpiece positioned directly above the compartment, due to the compartment being covered. The release mechanism is, preferably, instead fluidly connected to a mouthpiece downstream of the release mechanism such that the medicament-laden air from a compartment first travels along the fluid conduit provided by the release mechanism covering the compartment, preferably across the width of the carrier strip, before it can then travel (or pass) to the mouthpiece.

Described is a device for dispensing discrete doses of medicament carried in a plurality of spaced-apart compartments on a medicament carrier disposed within the device, the device comprising: a housing having a mouthpiece for inhalation; a release mechanism for releasing medicament from a compartment, the release mechanism being arranged to engage with the medicament carrier and release the medicament by at least partially exposing the compartment; wherein the release mechanism is fluidly connected with the mouthpiece such that the medicament can be dispensed from a compartment that is at least partially exposed during engagement of the release mechanism with the medicament carrier.

As noted above, the release mechanism preferably covers said exposed compartment so as substantially to seal the compartment in place of the sealing layer such that air can only pass through (e.g. or enter) said compartment via the release mechanism. The release mechanism may be an integral part of an air flow path that fluidly connects the release mechanism to a mouthpiece. In other words, the mouthpiece is preferably downstream of the release mechanism, wherein the air flow path is substantially a "closed" air flow path (similar to a closed conduit, rather than an "open" channel, for example). The air flow path may be across the travel path of the medicament carrier, for example out of the plane of said travel path, such that air flowing along the air flow path passes through (e.g. into) the exposed portion of the compartment.

The release mechanism may be arranged to pass air through an at least partially exposed compartment so as to aerosolise the medicament dose carried therein.

The device may further comprise a bypass air path arranged to fluidly connect with the air flow path downstream of the release mechanism, wherein air passing along the bypass air path does not pass through the release mechanism.

According to another aspect of the present invention there is provided a device for dispensing discrete doses of medicament carried in a plurality of spaced-apart compartments on a medicament carrier disposed within the device, the device comprising: a housing having a mouthpiece for inhalation; a release mechanism for at least partially exposing a compartment to an air flow path fluidly coupled between the release mechanism and the mouthpiece; and a bypass air path arranged to fluidly connect with the air flow path downstream of the release mechanism, wherein air passing along the bypass air path does not pass through the release mechanism.

As noted above, the release mechanism preferably covers said exposed compartment so as substantially to seal the compartment in place of the sealing layer such that air can only pass through (e.g. or enter) said compartment via the release mechanism.

The bypass air flow may comprise means for controlling the flow of air through the bypass air flow during inhalation. The release mechanism may comprise means for controlling the flow of air through the compartment during inhalation. The device may be configured such that the air resistance through the release mechanism is greater than the air resistance of the bypass air path.

The device may further comprise a second release mechanism for engaging with a second medicament carrier disposed in the housing, the second release mechanism being fluidly connected with the mouthpiece such that a dose of medicament carried by the second medicament carrier can be dispensed during engagement of the second release mechanism with the second medicament carrier.

Described is is provided a device for dispensing discrete doses of medicament carried in a plurality of spaced-apart compartments on first and second medicament carriers disposed within the device, each compartment being arranged in a carrier strip and sealed by a sealing layer, the device comprising: a housing having a mouthpiece for inhalation; a first release mechanism for engaging with the first medicament carrier, the first release mechanism being fluidly connected with the mouthpiece such that a dose of first medicament can be dispensed during engagement of the first release mechanism with the first medicament carrier; and a second release mechanism for engaging with the second medicament carrier, the second release mechanism being fluidly connected with the mouthpiece such that a dose of second medicament carried by the second medicament carrier can be dispensed during engagement of the second release mechanism with the second medicament carrier.

The first and second release mechanisms may be integral parts of, preferably separate, first and second air flow paths that fluidly connect to the mouthpiece. The first and second flow paths may be arranged to combine air flow streams downstream of the respective first and second release mechanisms. The two separate air flow streams may be combined within the device, or kept separate until they have exited the device. Preferably, the first and second flow paths are kept separate within the device such that the two separate air flow streams combine downstream of the mouthpiece (i.e. external of the device), for example in the mouth of a user during inhalation from the device.

The device may further comprise first and second bypass air paths as described above, wherein the first bypass air path may be arranged to fluidly connect with the first air flow path downstream of the first release mechanism, and wherein the second bypass air path may be arranged to fluidly connect with the second air flow path downstream of the second release mechanism. The bypass air paths may fluidly connect with the first and second flow paths, respectively, within the device, even if the two air flow streams are kept separate until exiting the device.

The first and second release mechanisms may be arranged to engage with the respective first and second medicament carriers at substantially the same time such that a mixture of the first and second medicament doses can be inhaled (e.g. through the mouthpiece) by a user of the device. Importantly, the first and second medicament doses may be released substantially simultaneously, such that they can be inhaled in combination by a user. A user will, of course, still inhale a mixture of the first and second medicament doses if they exit the mouthpiece separately but mix immediately thereafter, e.g. during inhalation in the mouth of the user.

The first and second release mechanisms may be substantially the same mechanisms. The first and second release mechanisms may be arranged in the housing adjacent each other, for example in a substantially orthogonal configuration. The first and second release mechanisms may be arranged in the housing substantially one above the other.

The device may further comprise a mixing chamber fluidly connected between the mouthpiece and the first release mechanism such that the medicament dose released by the first release mechanism passes through the mixing chamber prior to reaching the mouthpiece during inhalation. Alternatively, the mouthpiece may comprise (e.g. be configured to function as) a mixing chamber whereby the aerosolised medicament dose passes directly from the first release mechanism to the mouthpiece.

According to another aspect of the present invention there may be provided a device for dispensing discrete doses of medicament carried in a plurality of spaced-apart compartments on a medicament carrier disposed within the device, each compartment being arranged in a carrier strip and sealed by a sealing layer, the device comprising: a housing having a mouthpiece for inhalation; a first release mechanism for engaging with the medicament carrier, the release mechanism being fluidly connected with the mouthpiece such that a dose of medicament can be dispensed during engagement of the release mechanism with the medicament carrier; a mixing chamber fluidly connected between the mouthpiece and the first release mechanism such that medicament dose released by the first release mechanism passes through the mixing chamber prior to reaching the mouthpiece during inhalation.

As mentioned above, alternatively, the mouthpiece may comprise (e.g. be configured to function as) a mixing chamber, whereby the aerosolised medicament dose passes directly from the first release mechanism to the mouthpiece.

The mixing chamber may be configured to de-aggregate aerosolised medicament in air entering the mixing chamber from the release mechanism. The mixing chamber may be arranged to generate a vortex in the air as it enters the mixing chamber. The mixing chamber may be arranged such that air enters the mixing chamber at an angle offset from the central axis of the mixing chamber.

The device may comprise a second release mechanism as described above, wherein the mixing chamber may be fluidly coupled to both the first and second release mechanisms such that first and second medicaments can be mixed together in the mixing chamber during inhalation prior to reaching the mouthpiece. Alternatively, the mixing chamber may be configured such that the first and second medicaments are kept separate within the device such that they only mix after exiting the mouthpiece, and hence the device. For example, the mouthpiece may comprise separate first and second mixing chambers, or a single mixing chamber that is be divided.

The first and second release mechanisms may be fluidly connected to the (or each) mixing chamber via first and second air flow paths.

The device may further comprise first and second bypass air paths, wherein the first bypass air path may be arranged to fluidly connect with the first air flow path downstream of the first release mechanism, and wherein the second bypass air path may be arranged to fluidly connect with the second air flow path downstream of the second release mechanism, wherein the first and second bypass air paths do not pass through the compartments, for example the first and second bypass air paths do not through the first and second release means. The first and second bypass air paths may therefore be configured to fluidly connect separately with the first and second air flow paths, respectively, for example within separate mixing chambers, which may be provided by the mouthpiece or separately downstream of the mouthpiece.

The term "bypass" air path as used herein preferably connotes any air path that does not pass through an exposed compartment to entrain or aerosolise a medicament dose contained therein.

The device may further comprise a vent located on an outer surface of the housing and fluidly connected to the mouthpiece via the release mechanism to provide an air flow path for medicament to be carried to the mouthpiece from a compartment at least partially exposed by the release mechanism. The vent may (in addition to being fluidly connected with the release mechanism) be fluidly connected to the air flow path downstream of the release mechanism via a bypass air path that bypasses the release mechanism, and preferably the compartment.

The device may further comprise a cover configured to move between a first configuration in which the mouthpiece in the device is covered and a configuration in which the mouthpiece is uncovered, wherein the vent is covered by the cover in both the first and second configurations.

According to another aspect of the present invention there is provided a device comprising: a housing; a vent configured to allow external air to enter the housing; a mouthpiece configured to allow a user to inhale air through the housing; a cover configured to move between a first configuration in which the mouthpiece is covered and a second configuration in which the mouthpiece is uncovered, wherein the vent is covered by the mouthpiece cover in both the first and second configurations.

The cover may be arranged to cover the vent closely when in the first configuration and to cover the vent loosely when in the second configuration.

The vent may be substantially airtight when the cover is in the first configuration and wherein the vent allows passage of fluid through it when the cover is in the second configuration.

The release mechanism may be arranged to separate (e.g. peel away) at least part of the sealing layer from the carrier strip thereby to expose the medicament dose contained therein to an air flow path that fluidly connects the exposed compartment to the mouthpiece. As noted above, the air flow path is preferably part of a "closed" fluid conduit formed by the release mechanism covering the exposed compartment of the carrier strip.

The device may further comprise an indexing mechanism arranged to advance the medicament carrier relative to the release mechanism, wherein the indexing mechanism comprises a rotatable drive element having a plurality of spaced-apart indentations arranged around its outer perimeter, each indentation being configured to receive a compartment of the medicament carrier such that rotation of the rotatable element causes the medicament carrier to advance; and the depth of each indentation is less than the depth of the compartments such that the medicament carrier is urged against the release mechanism as it is advanced.

The carrier strip may be urged against the release mechanism so as substantially to seal an exposed portion of the compartment. The device may further comprise a biasing means arranged to urge the medicament carrier against the release mechanism. The device may further comprise a guide means for the carrier strip to advance along, the guide means being configured to urge the compartments of the medicament carrier against the release mechanism.

The device may further comprise means for engaging with a portion of the sealing layer of a medicament carrier as it passes through the release mechanism such that contact is maintained between the sealing layer and the carrier strip as the medicament carrier passes through the release mechanism, preferably wherein the means for engaging is arranged to engage with a substantially central portion of the sealing layer.

The device may further comprise at least one rotatable element arranged to receive and retain together, by way of rotation, the separated portions of carrier strip and sealing layer of the or each medicament carrier.

According to another aspect of the present invention there is provided a device for releasing discrete doses of medicament carried in a plurality of spaced-apart compartments on at least one medicament carrier, each compartment being arranged in a carrier strip and sealed by a sealing layer, the device comprising: at least one release mechanism for releasing medicament from a compartment by separating at least a portion of the sealing layer from the carrier strip to expose the medicament contained therein; and at least one rotatable element arranged to receive and retain together, by way of rotation, the separated portions of carrier strip and sealing layer of the (or, optionally, each) medicament carrier.

The rotatable element may be arranged to receive and retain together, downstream of the release mechanism, two separated carrier strips and sealing layers from two separate medicament carriers, for example on a spindle, preferably on two separate spindles.

According to another aspect of the present invention there is provided a device for releasing discrete doses of medicament carried in a plurality of spaced-apart compartments on first and second medicament carriers disposed within the device, each compartment being arranged in a carrier strip and sealed by a sealing layer, the device comprising: a first release mechanism for releasing a discrete dose of medicament from a compartment of a first medicament carrier; and a second release mechanism for releasing a discrete dose of medicament from a compartment of a second medicament carrier, wherein the first and second release mechanisms are each configured to release discrete doses medicament from a compartment of the first and second medicament carrier, respectively, by separating at least a portion of the sealing layer from the carrier strip to expose the medicament contained therein; and a rotatable element arranged to receive and retain together, by way of rotation, the separated portions of sealing layer of the two medicament carriers.

The device is preferably configured to release a discrete dose of medicament from a compartment of each medicament carrier substantially simultaneously.

The separated carrier strip(s) and/or sealing layer(s) may not be held substantially under tension on the rotatable element. For example, while the rotatable element is preferably driven to take up (e.g. wind up) the portions of the medicament carrier that have been separated, and may therefore apply tension to do so, it is not necessary for those portions to be stored on the rotatable element under tension. Alternatively, or additionally, the separated portions of sealing layers may be held under tension, for example to help with the advancement of the medicament carriers through the respective release mechanisms.

Described is a method of releasing discrete medicament doses carried in a plurality of spaced-apart compartments in a medicament carrier, each compartment being arranged on a carrier strip and sealed by a sealing layer to form a medicament carrier, the method comprising: separating at least a portion of the sealing layer from the carrier strip such that a medicament dose contained in a compartment is exposed; releasing at least a portion of the medicament dose from the compartment; bringing back together the separated portion of the sealing layer; and storing the separated sealing layer and carrier strip together on a rotatable member such that the separated sealing layer and carrier strip are wound together around said rotatable member.

Described is a method of releasing discrete medicament doses carried in a plurality of spaced-apart compartments on first and second medicament carriers, each compartment being arranged in a carrier strip and sealed by a sealing layer, the method comprising: separating at least a portion of the sealing layer from the carrier strip of each medicament carrier such that a discrete medicament dose contained in a compartment of each carrier strip is exposed; and storing the separated sealing layers together on a rotatable member such that the separated sealing layers are wound together around said rotatable member.

The separating step may further comprise exposing the discrete medicament dose in a compartment of each medicament carrier substantially simultaneously. The separating step may comprise inserting an element (or member), for example of a release mechanism, at least partially between the sealing layer and the carrier strip and moving the medicament carrier relative to the inserted element so as to separate at least a portion of said sealing layer from said carrier strip such that a medicament dose contained in a compartment is exposed. While the medicament dose may be exposed, it may still be contained by the element (or member), which may further form part of a fluid conduit, for example, rather than being free to fall out of, or be spilled from, the compartment should the device be moved.

The discrete doses of medicament are preferably released from a respective compartment of each medicament carrier substantially simultaneously.

Described is a method of releasing discrete medicament doses carried in a plurality of spaced-apart compartments in a medicament carrier, each compartment being arranged on a carrier strip and sealed by a sealing layer to form a medicament carrier, the method comprising: engaging (or inserting) a release mechanism at least partially between the sealing layer and carrier strip; and moving the medicament carrier relative to said engaged (or inserted) release mechanism so as to separate at least a portion of said sealing layer from said carrier strip such that a medicament dose contained in a compartment is exposed.

The separating step may further comprise completely separating the sealing layer from the carrier strip in the region of a compartment such that substantially the entire compartment is exposed.

Releasing the medicament dose may comprise arranging a partially exposed compartment to be in air flow path that is fluidly connected to a mouthpiece for inhalation such that air flowing along the air path collects, and preferably aerosolises, the medicament dose in the compartment as the air passes through or over it, preferably during inhalation.

Again, while the medicament dose may be exposed, it may still be contained by the release mechanism, which may further form part of a fluid conduit, for example, rather than being free to fall out of, or be spilled from, the compartment should the device be moved.

Described is a method of releasing discrete medicament doses carried in a plurality of spaced-apart compartments in a medicament carrier, each compartment being arranged on a carrier strip and sealed by a sealing layer to form a medicament carrier, the method comprising: removing, using a release mechanism (e.g. the devices described herein), at least a portion of the sealing layer covering a compartment to expose the medicament dose contained therein; and passing air through or over the compartment, via the release mechanism, to aerosolise said medicament dose.

The method may further comprise covering a portion of the compartment from which said at least a portion of the sealing layer has been removed with (at least part of) the release mechanism, wherein said (at least part of the) release mechanism is arranged to form (e.g. an integral) part of an air path through which air is passed. Thus, while the medicament dose may be exposed, it may still be contained by the release mechanism, which may further form part of a fluid conduit for the air path, for example, rather than being free to fall out of, or be spilled from, the compartment should the device be moved.

According to another aspect of the present invention there is provided an elongate medicament carrier comprising a plurality of spaced-apart compartments on a carrier strip, each compartment being sealed by a sealing layer, wherein a portion of the carrier strip comprises at least one pocket where the carrier strip is not attached to the sealing layer arranged on one or both sides of the medicament carrier, the at least one pocket preferably extending across the width of the carrier strip for receiving a release mechanism configured to separate the sealing layer from the carrier strip.

The portion of the carrier strip may comprise a plurality of said pockets, preferably arranged on both sides of the medicament carrier, and preferably being opposed if not passing through the entire width of the medicament carrier.

Alternatively, the release mechanism may comprise at least one elongate element disposed between the carrier strip and the sealing layer such that the application of tension to the at least one elongate element causes it to lift away from the carrier strip and tear the sealing layer to at least partially expose the medicament dose contained in a compartment. A portion of the elongate element may not be sealed between the compartment and the sealing layer to provide for the application of tension. The elongate element may be disposed between the carrier strip (e.g. and the compartments) and the sealing layer, and arranged such that the application of tension on the wire element causes it to lift away from the compartment and tears the sealing layer to expose the medicament dose contained therein

Indeed, a medicament carrier may therefore comprise a plurality of spaced-apart compartments on a carrier strip, each compartment being sealed by a sealing layer, wherein the carrier strip further comprises at least one elongate element disposed between the carrier strip (e.g. and the compartments) and the sealing layer, and arranged such that the application of tension on the at least one elongate element causes it to lift away from the compartment and tear the sealing layer over a compartment to expose the medicament dose contained therein. In other words, the tear to the sealing is localised for each successive compartment from which the medicament doses are released. The elongate element may be a wire.

According to another aspect of the present invention there is provided a device for releasing discrete doses of medicament carried in a plurality of spaced-apart compartments on a medicament carrier, each compartment being arranged in a carrier strip and sealed by a sealing layer, the device comprising means for applying tension to a first portion of a carrier strip and a second portion of a carrier strip, the sealed compartment located between the first and second portions, such that moving the first portion away from the second portion at least partially unseals the compartment.

According to another aspect of the present invention there is provided a medicament carrier, the carrier comprising a plurality of spaced-apart compartments each carrying a discrete medicament dose, wherein the compartments are arranged such that the application of tension to the carrier on either side of each compartment causes the compartment to be at least partially opened.

The medicament carrier may comprise two identical strips of elongate carrier material that are attached together at regular intervals in a parallel arrangement along the length of the carrier such that unattached portions of the strips of carrier material are provided between attached portions of the strips of elongate carrier material, wherein the respective strips of carrier material on either side of an unattached portion of carrier are folded or concertinaed onto themselves to form sealed compartments containing the discrete doses of medicament, such that the application of tension to the attached portions located immediately on either side of a sealed compartment causes the compartment to at least partially unseal thereby releasing the dose of medicament.

The compartment may be further arranged to be opened by shaped element arranged to fit between the concertinaed portions thereby to prise apart the attached portions once tension has been applied to at least partially unseal the compartment, preferably after the compartment has been initially opened, at least partially, by pulling apart the concertinaed potions, for example by applying tension to the medicament carrier on either side of the compartment.

The medicament carriers referred to herein are, preferably, elongate.

As will be recognised by a skilled person, numerous advantages over the prior art are provided by the various inventive concepts disclosed herein. For example, by combining the functions of certain components and simplifying the release mechanism, a medicament delivery device can be produced which comprises fewer internal components and, consequently, is less complicated and less expensive to manufacture.

Furthermore, it will be understood by the skilled person that any feature described in relation to a particular aspect herein may also be applied to another aspect described herein, in any appropriate combination. It will also be appreciated that particular combinations of the various features described and defined in any aspects described herein can be implemented and/or supplied and/or used independently.

In addition, any apparatus feature described herein may be provided as a method feature, and vice versa. Furthermore, as used herein, means plus function features may be expressed alternatively in terms of their corresponding structure. Moreover, it will be understood that the present invention is described herein purely by way of example, and modifications of detail can be made within the scope of the invention.

As used herein, the terms upstream and downstream preferably connote positions in the air flow path relative to the release mechanism, for example wherein upstream of the release mechanism is before to release of the medicament and downstream of the release mechanism is after release of the medicament. Also, as used herein, the terms flowpath and passageway may be interchangeable.

In the following description and accompanying drawings, corresponding features of different embodiments may be identified using corresponding reference numerals.

### Brief Description of the Drawings

Embodiments of the present invention will now be described, by way of example only, with reference to the accompanying figures, in which:
Figure 1 shows a cross-sectional view of a known multi-unit dose dry powder inhaler device;
Figures 2A to 2C show a device according to a first aspect;
Figures 3A to 3C shows a device according to a second aspect;
Figure 4 shows a multi-unit dose medicament carrier for use with the devices of Figures 2 and 3;
Figures 5 and 6 show alternative multi-unit dose medicament carriers;
Figures 7A to 7C show three different internal configurations for a medicament delivery device;
Figures 8A and 8B show a medicament carrier engaged by devices shown in

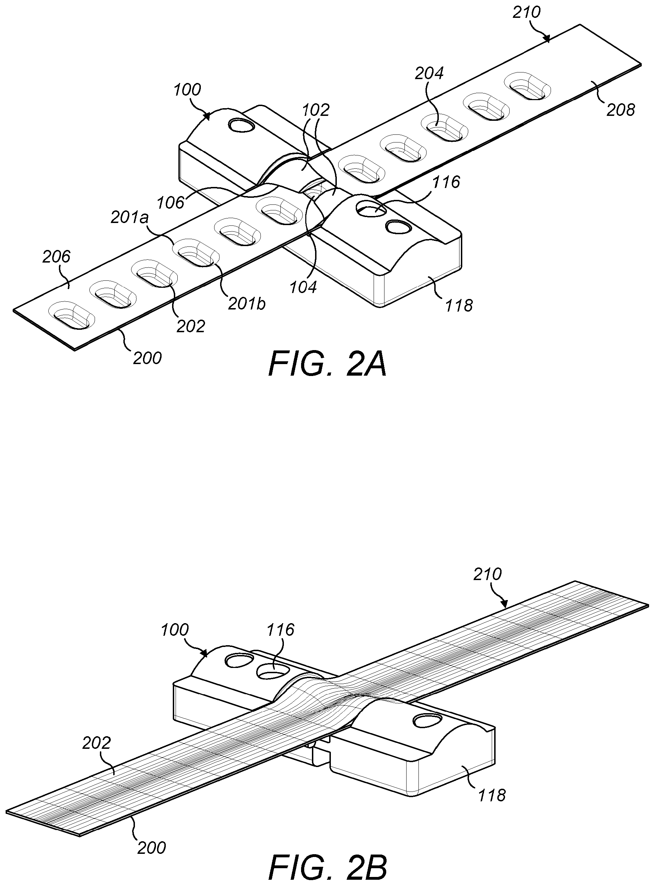

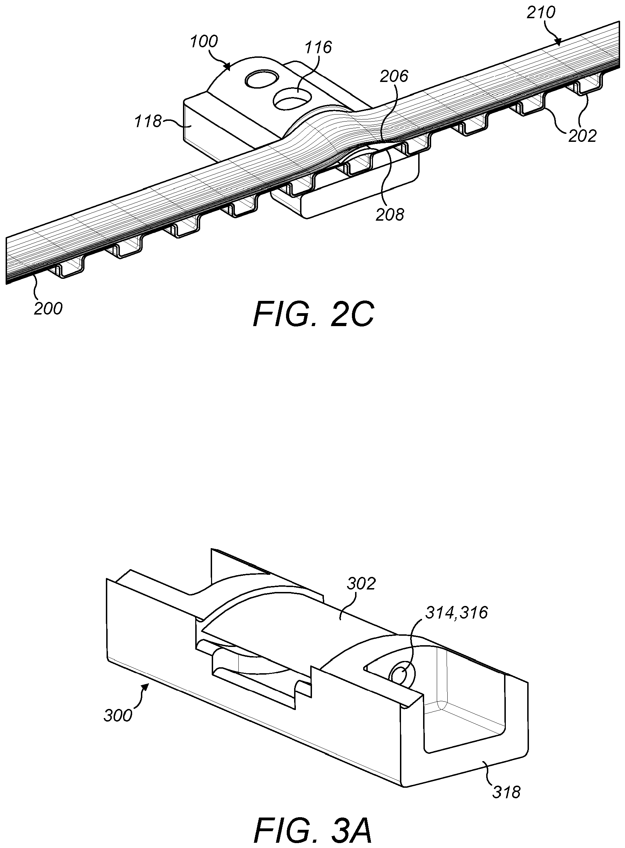

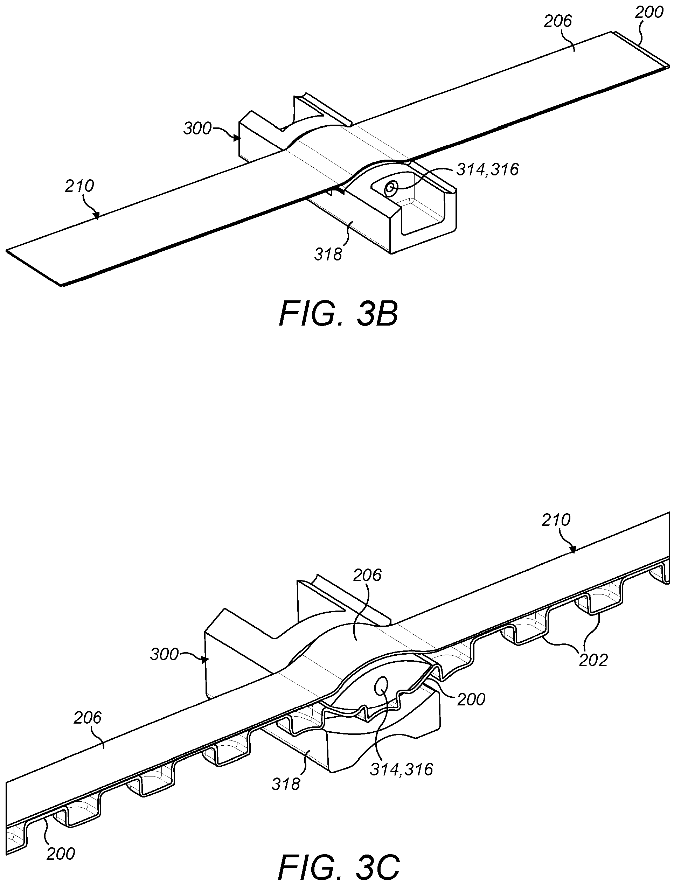

Figures 2 and 3, respectively;

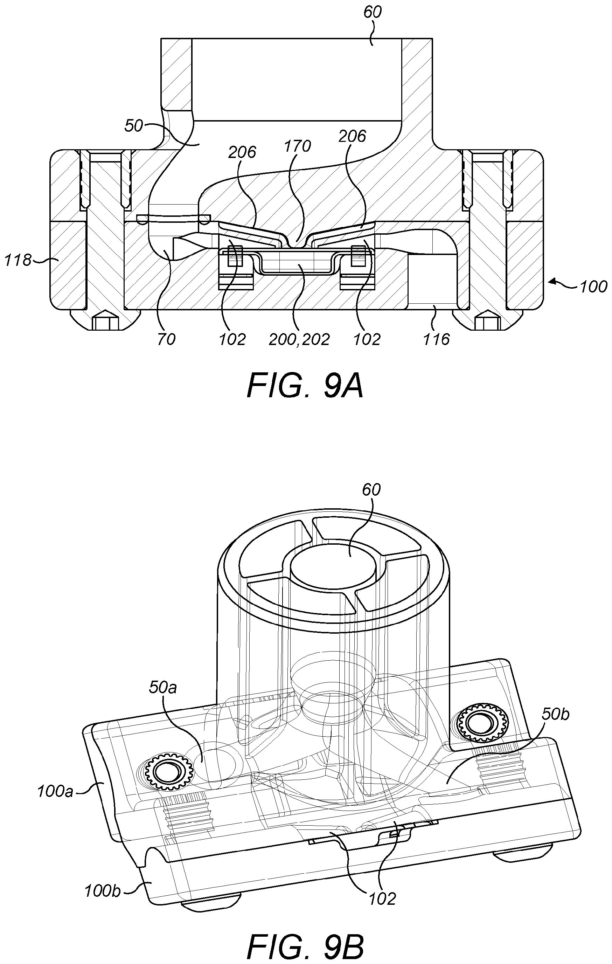

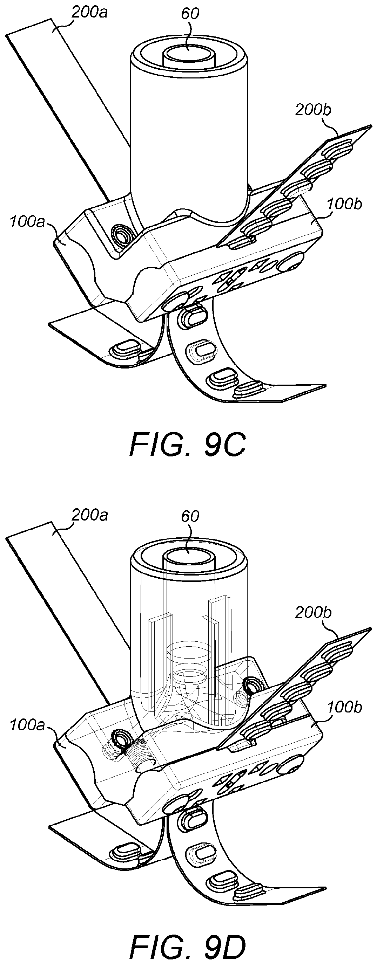

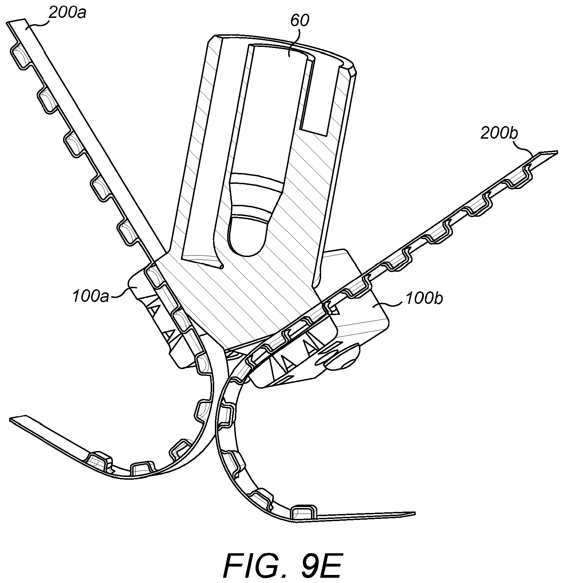

Figures 9A to 9E show two devices fluidly connected with a mixing chamber; and

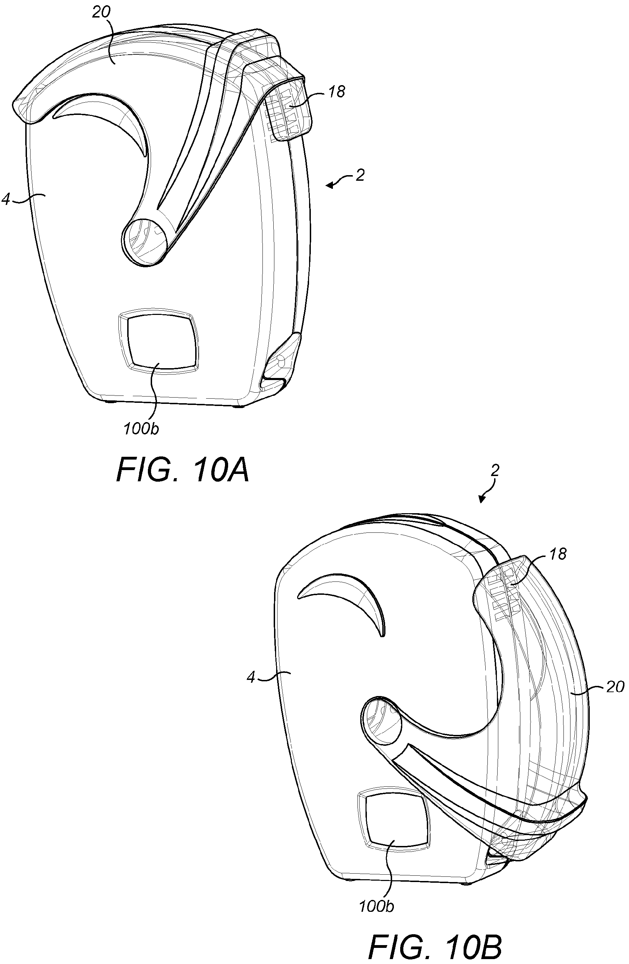

Figures 10A and 10B show a medicament delivery device with a cover located in first and second configurations.

### Detailed description

It is increasingly common for inhaled drug therapies to use combinations of two dry powder formulations which are stored separately and then mixed at the time the user is ready to inhale the drug. Thus, a medicament delivery device may be required to dispense two different medicaments, which are preferably combined immediately prior to inhalation so that when a user inhales through a mouthpiece, they inhale a mixture of the two dry powder formulations.

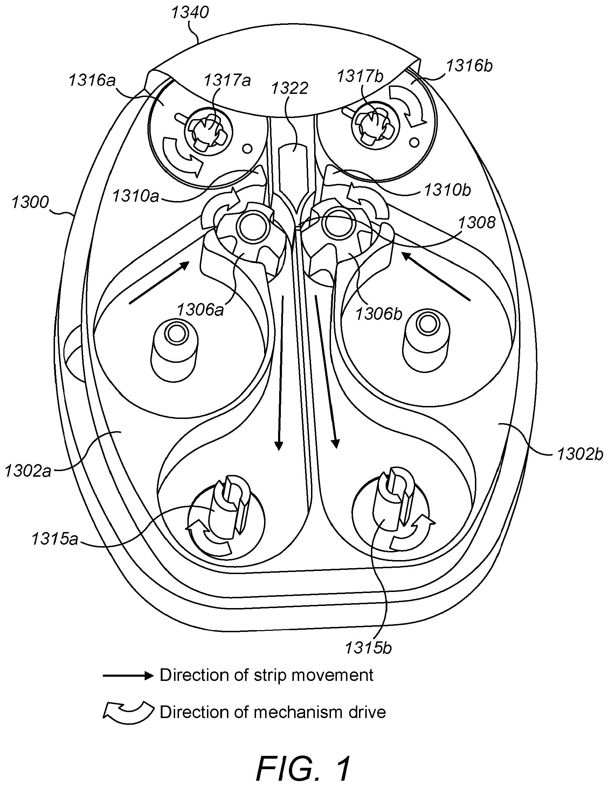

A known dry powder inhaler (DPI) device that can dispense a mixture of two dry powder formulations is disclosed in WO 2003/061743, an embodiment of which is illustrated in Figure 1. Once fully assembled, this DPI device 1300 will contain two medicament carriers (not shown - but described below), each comprising a plurality of spaced-apart, sealed compartments and each stored on one of the delivery spools in the centre of the device housing.

Rotatable drums 1306a, 1306b are operable to engage each medicament carrier via its compartments so as to advance the next compartments towards the opening station 1322 when a medicament dose is required. The two medicament carriers contain different powder formulations, which are released at opening station 1322 where they are intended to mix together immediately prior to inhalation.

In use, the compartments containing the medicament doses on each carrier strip are opened in turn by gradually peeling away the sealing layer to release the medicament dose contained in each compartment. The peeling action occurs at peeling stations 1310a, 1310b, where the sealing layer is gradually peeled away by pulling the sealing layer from the carrier strip in a generally orthogonal direction. This is achieved by the application of tension to the sealing layer by a release mechanism comprising a pair of spools 1317a, 1317b to which an end of the sealing layer is secured. As the medicament carrier advances to the next compartments, the "release" spools 1317a, 1317b rotate to gather the peeled sealing layer while the portion of the carrier strips comprising the exposed (or "opened") compartments remain engaged with the rotatable drums 1306a, 1306btemporarily before being gathered around a pair of "take-up" spools 1315a, 1315b disposed remotely from the "release" spools 1317a, 1317b elsewhere in the device.

A typical medicament carrier 210, as is well-known, is shown in Figure 2A, which comprises a carrier strip 200 that includes a plurality of spaced-apart compartments 202 and a sealing layer 206, which is shown as transparent in Figure 2A to aid understanding. Each compartment 202 comprises a discrete (or "unit") medicament dose. The carrier strip 200 is an elongated strip with the compartments 202 being equally spaced along the length of the carrier strip 200. The compartments 202 are substantially elongated across a portion of the width of the carrier strip 202 and have an opening 204 which is substantially flush with a top surface 208 of the carrier strip 200. The compartments 202 are generally lozenge-shaped, but could be other shapes, such as round or rectangular, for example. The sealing layer 206, which may also be referred to as a "backing sheet", seals the compartments 202. Each medicament dose is thereby securely contained within its compartment 202, preventing contamination of the medicament dose. The sealing layer 206 is usually secured to the top surface 208 of the carrier strip 200, typically covering substantially the whole of the carrier strip 200. Such medicament carriers may also be known or referred to as "blister strips".

An example of an improved release mechanism 100, for releasing medicament doses contained in compartments of a conventional medicament carrier, is shown in Figures 2A-2C. The medicament may, of course, comprise a dry powder formulation.

Figures 2A-2C show the release mechanism 100 engaging with such a "conventional" medicament carrier 210. For engaging with the medicament carrier 210, the release mechanism 100 includes two release members 102 arranged substantially opposite each other such that they extend towards each other. In this release mechanism 100, a gap 104 is left between the two release members 102, as can be seen in Figure 2A. The release members 102 are housed in a body 118 of the release mechanism 100.

Each release member 102 has a lower surface 106 arranged to pass closely over the top of the carrier strip 200 and an upper surface 108 having a leading edge 110 arranged to engage between the carrier strip 200 and the sealing layer 206 and thereby effect separation. In the arrangement shown, the lower surface 106 is substantially flat and the upper surface 108 is a curved surface having a curved leading edge 110. However, the lower surface 106 may alternatively be a curved surface and the upper surface is a substantially flat surface having a curved leading edge 110. In some arrangements, both the lower and upper surfaces could be curved or, alternatively, both the lower and upper surfaces could be substantially flat, or have any other suitable profile to effect the desired separation.

The release members 102 are configured to allow the optimum removal (i.e. separation) of sealing layer 206 from the carrier strip 200 while ensuring that the release members 102 do not overlap two consecutive compartments 202 along the carrier strip 200 at the same time.

In the arrangement in Figure 2, the release members 102 have a generally tapered configuration, where the release members 102 taper inwardly. The leading edge 110 of the release member 102 (i.e. the edge that first engages the sealing layer 206, in use) is arranged to conform to the shape of a portion of the perimeter of the compartment 202 such that the release member 102 can remove the maximum amount of sealing layer 206 from an intended compartment 202 without removing any sealing layer 206 from the next compartment 202 on the carrier strip 200. Similarly, the trailing edge of the release member 102 is arranged to conform to the shape of a portion of the perimeter of the compartment 202 such that the release member 102 does not overlap a previously exposed compartment 202 when the release mechanism 100 is engaging a new, sealed compartment 202. Other configurations of the release members are possible.

As the carrier strip 200 is fed through the release mechanism 100, the release members 102 engage the medicament carrier 210 between the sealing layer 206 and carrier strip 200 so that the carrier strip 200 passes beneath the release members 102 and the sealing layer 206 passes over the leading edge 110 of the release members 102. This engagement causes at least a portion of the sealing layer 206 to become separated (i.e. removed) from the carrier strip 200, thereby exposing at least a portion of the compartment 202 and the medicament dose contained therein, as shown in Figure 2C.

As is shown in Figures 2A and 2B, the release members 102 do not extend across the width of the carrier strip 200. This has the effect that the release members 102 only separate the sealing layer 206 from the carrier strip 200 across the two end portions 201a, 201b (or "sides") of each compartment 202 as the carrier strip 200 advances through the release mechanism 100.

With this release mechanism 100, a central part of the sealing layer 206, which covers a central portion of the compartments 202 and carrier strip 200, remains attached to the carrier strip 200. This can help to retain any medicament that remains in a compartment 202 after the dose has been dispensed from falling out of the compartment 210 into a surrounding housing (not shown).

The central portion of the sealing layer 206 may be urged against the carrier strip 202 as the medicament carrier 210 passes through the release mechanism 100. For example, a protuberance 170 may extend into the space 104 between the release members 102, as can be seen in Figure 9A. The protuberance 170 is arranged to contact the sealing layer 206 and thereby retain the sealing layer 206 in attachment with the (top surface of the) carrier strip 200.

On the underside of each of the release members 102 may be provided an aperture 114, shown in Figure 2C, for air to be blown through the release members 102 and into a compartment. Furthermore, each of the release members 102 may comprise an internal fluid conduit connected between the opening 114 and an aperture 116 on the body of the mechanism 100, shown in Figures 2A and 2B.

The two apertures 116 on the body of the mechanism 100 can be arranged such that, one aperture 116 is provided on an upper surface (e.g. on top) of the body on one side of the release mechanism 100, and another aperture 116 is provided on a lower surface (e.g. underneath) of the body on the other, opposite side of the release mechanism 100. As such, the release members 102 may form part of an air path way, and when the release members substantially cover the exposed portions on either side of a compartment 202 as it passes through the release mechanism 100 (i.e. with the remaining portion of the compartment 202 still sealed by the sealing layer 206), air can be blown through a substantially sealed compartment 202 via the internal fluid conduits of the opposed release members 102, to aerosolise the medicament contained within the compartment 202. This arrangement can therefore be used to remove medicament doses from a compartment 202, in use. This will be discussed in more detail further on.

Figures 3A-3C show an alternative release mechanism 300 to that shown in Figures 2A-2C. Instead of two opposed release members, the release mechanism 300 shown in Figure 3A comprises a single release member 302 arranged to extend across the full width of the carrier strip 200 and compartments 202, so that there is no gap between them. Similar to the previously described release mechanism 100, as the carrier strip 200 is fed through the release mechanism 300, the carrier strip 200 passes beneath the release member 302 and the sealing layer 206 passes over the leading edge 308 of the release member 302, as can be seen in Figure 3B. The sealing layer 206 is therefore completely separated from the carrier strip 200 fully exposing each compartment 202 as it passes the release mechanism 300, and hence the medicament dose contained therein, as shown in Figure 3C. The release member 302 is housed in a body 318 of the release mechanism 300.

Similar to the release members 102 of the previously described release mechanism 100, the single release member 302 may be arranged to pass air through an unsealed compartment 202 when engaged by the release mechanism 300. The release member 302 may therefore have one or more apertures (not shown) on the underside of the release member 302 each aperture being fluidly connected to an internal fluid conduit 316 that passes through the release member 302, as shown in Figures 3A and 3B. As such, when the release member 302 is substantially covering an unsealed compartment 202 that passes through the release mechanism 300, air can be blown through a substantially sealed compartment 202 via the internal fluid conduits 315 of the release member 302, to aerosolise the medicament contained within the compartment 202. This arrangement can also therefore be used to remove medicament doses from a compartment 202, in use. This will be discussed in more detail further on.

It is noted that Figures 3A and 3B may show slightly different versions of a release mechanism 300 according to this "alternative aspect", but the skilled person will recognise that the inventive concept is the same.

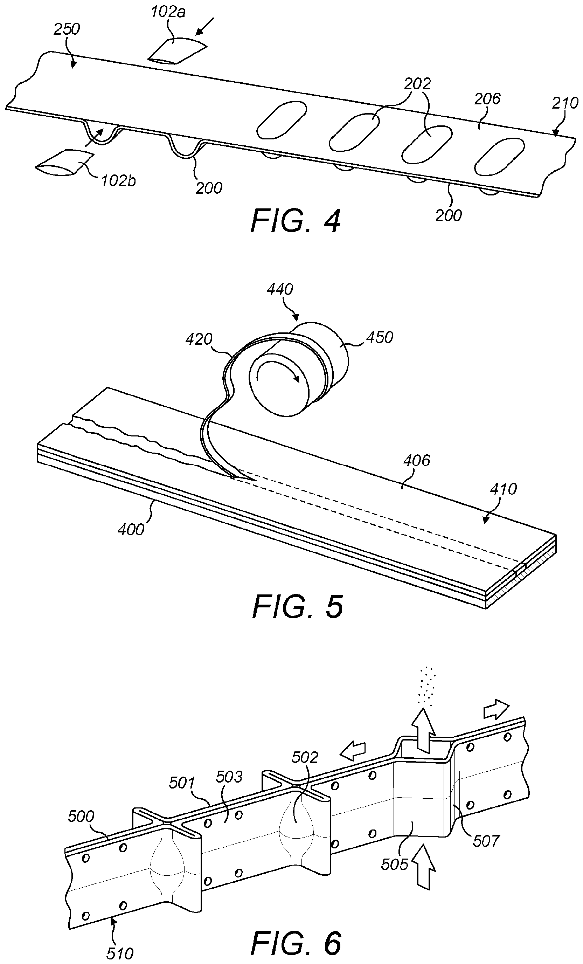

As shown in Figure 4, to facilitate engagement of the release member(s) 102, 302 with the medicament carrier 210 during assembly, a leading portion 250 of the carrier strip 200 may be free of sealed compartments 202 and instead comprise one or more pockets 260 (e.g. a portion where the sealing layer 206 is not attached to the carrier strip 200, possibly across the entire width of the carrier strip 200). The pockets 260 may be constructed in substantially the same way as the compartments 202 in that they are defined by a portion of the carrier strip 200 which is not attached to the sealing layer 206. However, in contrast to the compartments 202, the pockets 260 are open at both ends for receiving a release member 102, 302. The pockets 260 may have an opening with a width (in the direction of travel) that is less than the width of the projecting elements 102, 302. The pockets 260 may pass through the entire width of the carrier strip 200, or they may be "blind" pockets 260, which do not.

During assembly of the medicament delivery device, the one or more projecting elements 102, 302 are inserted into the pockets 260 so that the one or more projecting elements 102, 302 are correctly positioned between the carrier strip 200 and sealing layer 206, and the carrier strip 200 is positioned centrally with respect to the one or more projecting elements 102, 302.

To engage the release mechanism 100 comprising two release members 102 with a carrier strip 200 during assembly of a device, one side of the pocket 260 on the carrier strip 200 may be placed over a first projecting element 102a on one side of the release mechanism. The other, opposed projecting element 102b may then be brought into engagement with the pocket 260 on the other side of the carrier strip 200 in a similar manner so that both release members 102a, 102b engage the carrier strip 200. The first and second release members 102a, 102b may then be coupled, or otherwise secured. For example, the release mechanism 100 may comprise a body having two separable and connectable parts.

Similarly, to engage a release mechanism 300 comprising a single release member 302 with a medicament carrier, the release mechanism 300 may comprise two separable parts, with the release mechanism attached to one of said parts. Alternatively, two release mechanisms similar to those described above may be provided which join together in the middle to provide the same "release" effect as a single release member, such that each half can be inserted through a side of the pocket 260 as described above. Once the carrier strip 200 has been inserted on the release member 302, the two halves of the release mechanism 100 can then be secured together.

An alternative medicament carrier 410 is illustrated in Figure 5 comprising an elongate element 420 sealed between the compartments (not shown) of the carrier strip 400 and the sealing layer 406. In this example, the elongate element 420 is a wire element. The elongate element 420 is, preferably, located substantially centrally along the length of the carrier strip 400, though it could be arranged to be off-centre.

In order to remove the sealing layer 406 from the compartments 402 of the carrier strip 400 to unseal the compartments 402, a release mechanism 430 applies tension to (e.g. a free end of) the wire element 420. This causes the wire element 420 to be pulled away from the main body of the carrier strip 400 so that the wire element 420 lifts away from the compartment 402. As the wire element 420 is moved away from the compartments 402, the wire element 420 tears through the sealing layer 406 which exposes the compartment and the medicament dose, as can be seen in Figure 4. In some embodiments, two or more elongate elements may be sealed between the compartment 402 and the sealing layer 406 so that when tension is applied to the two or more elongate elements 420, the sealing layer 406 tears in multiple locations. The wire element(s) 420 can be collected on a further spool 450 as it becomes removed from the carrier strip 400. A release member, or similar, can then be arranged to pass air through the compartment, via the torn portion(s) to remove the medicament contained therein, similar to what has been described above. Sealing elongate members 420 between the carrier strip 400 and the sealing layer 406 does not compromise the moisture protection and offers a very simple way to open slits or tears in the sealing layer 400, which can then be fluidly connected to an air path way for release of the medicament.

Another alternative medicament carrier 510 is illustrated in Figure 6. Instead of using a combination carrier strip and sealing layer, the carrier strip 500 comprises first and second sheets 501, 503 that are attached together at regular intervals along the length of the carrier strip 500 in a parallel arrangement. Compartments 502 are formed along the length of the carrier strip 500 at locations where the two sheets 501, 503 are not attached to each other. That is, the unattached portions of the two sheets 501, 503 define the compartments 502 in which the medicament dose is contained. As is clear from Figure 6, the carrier strip 500 therefore comprises two sheets 501, 503 which are folded or concertinaed onto themselves to form the sealed compartments 502.

To unseal the compartments 502 and expose the medicament dose contained within the compartments 502, the release mechanism (not shown) applies tension (in the opposed directions shown by the arrows) to two attached portions 505, 507 of the carrier strip 500 either side of the compartment 502. The release mechanism 550 pulls the two attached portions 505, 507 in substantially opposing directions which causes the sealed compartments 502 to be pulled open, releasing the medicament dose. The first attached portion 505 is therefore moved away from the second attached portion 507 through the application of tension of each attached portion 505, 507.

In use, the folded, or concertinaed, carrier strip 500 is indexed into position in the release mechanism with the join between the two sheets 501, 503 being in vertical alignment with a fluid passageway (shown by the vertical arrows running in the same direction). The release mechanism grips the carrier strip 500 on either side of the compartment 502 and pulls the folded portion to unseal and expose the medicament dose to the air flow passageway.

As mentioned previously, drug therapies may require two separate medicament doses to be mixed together before being inhaled by a user. The doses are typically stored separately in the device and mixed within the medicament delivery device just before, or sometimes even during, inhalation by a user through a mouthpiece. A medicament delivery device may therefore contain two medicament carriers of similar construction from which medicament doses must be released. Such an arrangement will, ideally, require two similar release mechanisms.

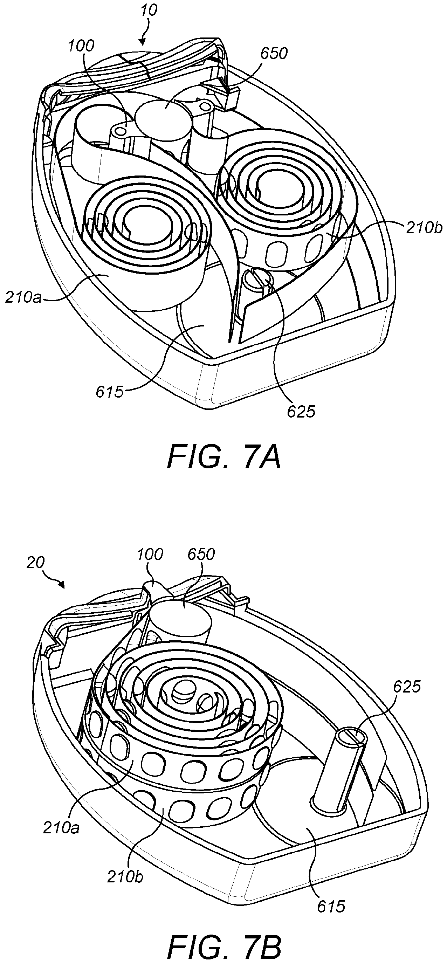

Figures 7A and 7B show two alternative configurations for medicament delivery devices 10, 20 for delivering separate medicament doses carried in separate medicament carriers 210a, 210b.

In the device 10 of Figure 7A, the medicament carriers 210a, 210b are stored on separate spools, with each medicament carrier 210 being arranged to feed through a respective release mechanism 100. The medicament carriers 210 are advanced by an indexing means, which comprises a rotatable element 650 having indentations spaced around it for receiving the compartments 202 on a carrier strip 200. The separated carrier strip 200 and sealing layer 206 of each medicament carrier 210 are retained together on a take-up spool 615, where they are wound together on a spindle 625 to gather up any slack. Preferably, to save space and components, the two medicament carriers 210a, 210b can be wound on the same take-up spool 615.

In an alternative embodiment (not shown), the separated portions of sealing layer 206 of each medicament carrier 210 may be retained together on a take-up spool, with the used portions of carrier strip 200 being stored on separate spools.

Alternatively, in order to reduce the number of parts required, the medicament carriers 210a, 210b may be stored on the same spool, as shown in the device 20 of Figure 7B. The spool may comprise opposing faces, arranged back-to-back, with each face having a centrally located spindle for mounting the medicament carriers 210a, 210b. Each medicament carrier 210a, 210b can then be wound onto its respective spindle (not shown).

As with the device 10 of Figure 7A, an end of each medicament carrier 210a, 210b here is fed through a release mechanism 100 suitable for unsealing the compartments of the carrier strips 200 and the ends of the medicament carriers 210a, 210b can then be attached to a "take-up" spool 615 arranged to retain and store the separated carrier strips and sealing layer of each medicament carrier 210a, 210b.

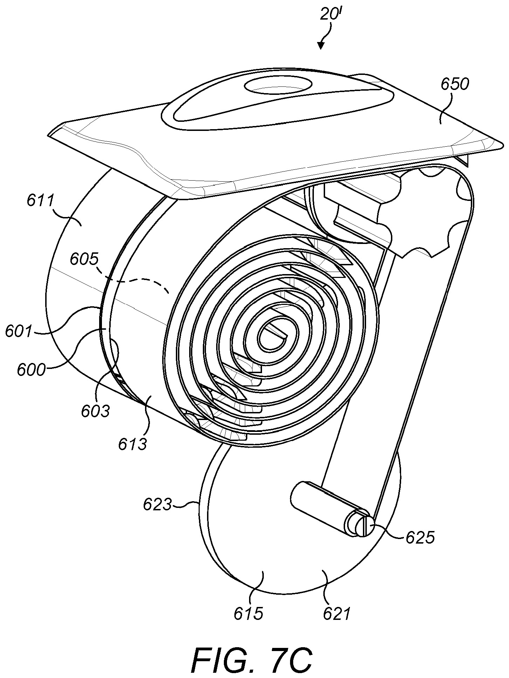

A device 20', having a configuration similar to the device 20 of Figure 7B, is shown in Figure 7C. The take-up wheel 615 of this device comprises opposed first and second faces 621, 623, each face having a spindle 625 to which the ends of the medicament carriers 210a, 210b are attached. Each face of the take-up wheel 615 stores both the main body of the carrier strip 200 comprising the compartments 202 and the sealing layer 206 of the carrier strip 200 after the carrier strip 200 has passed through the release mechanism 100 and the medicament dose been exposed within the compartments 202.

Arranging the two medicament carriers 210a, 220b close together (e.g. next to each other) makes the internal mechanism of the medicament delivery device 20' simpler because only the parts required for a single medicament carrier 210 are present. That is, the medicament delivery device does not require two sets of the same components, one for each medicament carrier 210. Instead, one set of components can be used with both medicament carriers 210a, 210b.

In order to feed the medicament carriers 210 through the release mechanism 100, the medicament delivery device may comprise an indexing mechanism 650 operatively connected to a drive means. As can be seen in the exemplary device 20' of Figure 7C, a suitable indexing mechanism 650 may comprise a rotatable drum 652 having a plurality of indentations 654 equally spaced around the external surface of the drum 652. The indentations 654 are shaped to receive a compartment 202 on a carrier strip 200. The drive means rotates the drum 652 which, in turn, causes the carrier strip 200 to be moved through the release mechanism 100. The indexing mechanism 650 is, preferably, positioned downstream of the release mechanism 100 so that the rotatable drum 650 pulls, rather than pushes, the medicament carrier 210 through the release mechanism 100. In each of these configurations, the release mechanism 100 could be immediately adjacent the indexing mechanism 650, such that the compartments 202 are opened during their engagement with the indexing mechanism 650.

It is important to make sure that the carrier strip 200 is positioned correctly relative to the release members 102 to ensure that the sealing layer 206 is correctly removed from the compartment 202 and a full medicament dose can be exposed to the air flow passageway. Furthermore, it is important to ensure that the release members 102 are correctly engaged with the medicament carrier 210, between the carrier strip 200 and the sealing layer 206.

Providing an indexing mechanism 650 with discrete indentations 654 in which the compartments 202 are received helps to ensure that each compartment 202 is in correct alignment with the release members 102. As the driving means causes the rotatable drum 652 to move in units of one indentation at a time, each compartment is fully aligned with the release member 102. The indexing mechanism 650 therefore avoids the likelihood that the carrier strip 200 will be fed through the release mechanism 100 in such a way that only part of the compartments 202 are aligned with the release members 102.

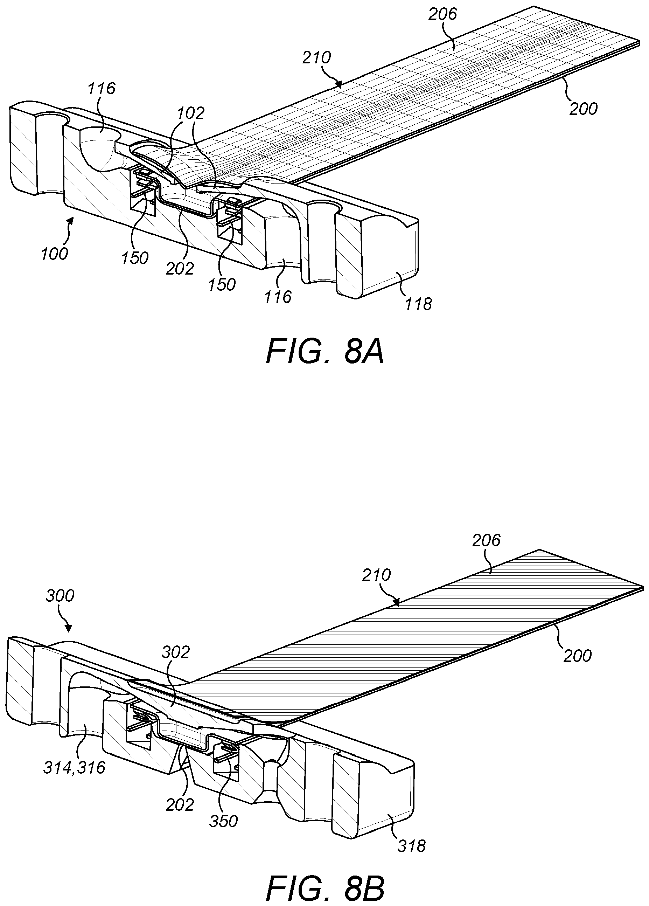

Figures 8A and 8B show release mechanisms similar to those of Figures 2 and 3, respectively, though with slightly different air flow path configurations that pass through the body of the release mechanisms 100, 300 rather than through the release members 102, 302.

Figure 8A shows a sectional view of the release mechanism 100 of Figure 2. The release members 102 cause portions of the sealing layer 206 to lift away from the carrier strip 200, thereby exposing the medicament contained in the compartment 202 to an air flow path that is configured to pass through the release mechanism 100, and hence the compartment 202, to aerosolise the medicament in the compartment and remove it via the air flow path.

Running the air flow path (or passageway) through the release members 102 means that when the carrier strip 200 has been unsealed either partially or fully, the carrier strip 200 does not need to be transferred anywhere before the medicament dose can be aerosolised. The air flow paths meet the compartment 202 outside of the plane of the carrier strip's path making it possible to have two carrier strips 200 cross the centreline of the medicament delivery device. This also makes it possible to stack the two carrier strips vertically, as shown in Figures 7B and 7C rather than have then positioned side-by-side, for example, as shown in Figure 7A, which may introduce constraints on the layout of the delivery device.

Similarly, Figure 8B shows a sectional view of the release mechanism 300 of Figure 3. The release member 302 separates the sealing layer 206 from the carrier strip 200 across the entire width, thereby exposing the medicament contained in the compartment 202 to an air flow path that is configured to pass through the release mechanism 300, and hence the compartment 202, to aerosolise the medicament in the compartment and remove it via the air flow path.

To reduce the chance of medicament dose being leaked into the internal mechanism of the delivery device, and to reduce an excess drop in air pressure due to leaks, the compartment 202 may be pressed firmly, or otherwise urged, against the lower surface 106 of the release members 102. This helps provide a substantially fluid-tight seal between the compartment 202 and the release member 102. This can be achieved by arranging the part of the release mechanism 100 through which the carrier strip 200 travels to have a height that is slightly less than the height of the compartments 202 of the carrier strip 200. This has the effect that when the carrier strip 200 is fed through the release mechanism 100, the compartments will be slightly compressed against the underside 106 of the release member 102.

Alternatively, the compartment 202 may be pressed firmly against the lower surface 106 of the release members 102 using a biasing means 150, 350 as shown in Figures 8A and 8B. The biasing means may, for example, be a spring arranged to urge the carrier strip 200 towards the release members 102.

In yet another arrangement (not shown) a guide may be provided for the carrier strip to run along, whereby the guide is configured to urge the carrier strip (and hence compartments) towards the release mechanism.

In medicament delivery devices in which two different formulations (i.e. carried in separate medicament carriers) are to be mixed, a mixing chamber 60 may be provided that is fluidly connected between a mouthpiece 16 for inhalation, and both release mechanisms 100, as shown in Figure 9A, such that the formulations entering the mixing chamber 60 from the respective release mechanisms 100 can be mixed together in the mixing chamber 60 prior to inhalation.

The sectional view of Figure 9A, further shows a release mechanism 100 engaged with a medicament carrier 210, showing the release members 102 covering exposed portions either side of a compartment 202, from which the sealing layer 206 has been removed. Once a particular compartment 202 in the carrier strip 200 has been opened, the medicament dose is exposed for release and to be aerosolised.

The structure of the air flow path can also be seen more clearly in Figure 9A.

In order to ensure that a full medicament dose is exposed for release from the compartment 202 into the chamber 60, it is important to minimise movement of the unsealed compartment 202 before the medicament dose has been exposed. The unsealing step is therefore combined with the release step ensuring that the maximum medicament dose can be transferred into the mixing chamber 60. To achieve this, the release members 102 may comprise part of a fluid passageway 50 (or "fluid conduit") that connects the chamber 60 with the compartment 202.

In such an arrangement, the lower surface 106 of the release members 102 may comprise an opening which is located above the compartment 202 of the carrier strip 200, as shown in Figure 8A. When the carrier strip 200 is fed into the release mechanism 100, the release member 102 engages between the top surface 208 of the carrier strip 200 and the sealing layer so that the opening on the lower surface 106 of the release member 102 is located above the unsealed portion of the compartment 202, as shown in Figure 8B. This opening leads into a passageway 70 through the release member 102 to join the main fluid passageway 50 which leads into the chamber 60. Thus, when the user draws air through the medicament delivery device via the mouthpiece, the air flows directly through the unsealed portion of the compartment 202, via the release member 102 and its passageway 70, collecting and aerosolising the exposed medicament in the compartment, then out through the fluid passageway 50, into the chamber 60, and then out through the mouthpiece.

Bypass air, for example air straight from a vent located externally on a housing of the device, can be introduced into the air streams downstream of the release mechanisms, which air streams include the exposed medicament dose from each compartment 202, before the diluted air streams are combined in the chamber 60 for de-aggregation. This allows the balance of the air which goes into each fluid passageway 50 to be controlled individually, independently from choosing the bypass-to-inlet (air) resistance ratio for a particular carrier strip 200 or combination of strips 200. This allows the fine particle fraction to be controlled much more precisely per drug without changing the overall flow resistance.

An important factor during aerosolisation of the medicament dose is the ratio between the air flowing through the compartment 202, which causes the medicament dose to be drawn out of the compartment 202 into the passageway 50, and the bypass air, which de-aggregates the medicament dose as it travels through the passageway 50 and fluid passageway 70.

Different medicament doses may have different compositions which results some medicament doses being more "sticky" than others. In this context, "sticky" refers to the ability of the powder forming the medicament dose to stick to itself. The stickier the powder of the medicament dose, the more air that is required to extract the medicament dose from the compartment 202 and transfer it through the passageways 50, 70.

Thus, the above-mentioned ratio can be adjusted (i.e. during design) to take into account the properties of the medicament dose. For example, the different fluid conduits or passageways for the "air flow path" (i.e. through the release mechanisms) and bypass air path (i.e. bypassing the release mechanisms) can be designed for a particular medicament (e.g. powder formulation) such that the air paths can be configured (e.g. during design) to be throttled relative to one another so as to provide a desired bypass-to-inlet (air) resistance ratio.

It is therefore important to be able to adjust the above-mentioned ratio in order to be able to extract a full medicament dose from the compartment 202. The ratio can either be adjusted up or down, i.e. the amount of air flowing through the compartment 202 can be increased or decreased relative to the amount of bypass air present in the passageways 50, 70. Adjusting this ratio ensures a good particle distribution can be obtained when the medicament dose is extracted from the compartment 202, preventing the user from inhaling clumps of medicament dose.

Figure 9B shows two release mechanisms 100a, 100b, both fluidly connected with a chamber 60 such that respective medicament doses released by each release mechanism 100a, 100b into the respective air flow paths 50a, 50b connecting with the chamber 60 will meet in the chamber 60, where they will become mixed prior to inhalation.

Figures 9C to 9E show various complementary views of the arrangement of Figure 9B, with two carrier strips 200a, 200b being engaged by the respective release mechanisms 100a, 100b. As discussed above, each release mechanism comprises passageways 50a, 50b for air to draw the medicament dose out of the compartments and into the chamber 60, as shown in Figures 9A and 9B.

As the medicament dose in each carrier strip 200 might have different properties, as explained above, it is possible to independently adjust the bypass air flows and/or the resistance of air flowing through the compartments 202 of each carrier strip 200a, 200b. Air flow can be adjusted by throttling the air flowing into the release mechanism, for example by configuring the narrowest parts of the release mechanism which comprises the air flow path according to the type of medicament dose present in the carrier strip 200. The bypass air path may be restricted in a similar manner, if required.

As previously mentioned, as the medicament delivery device may comprise two carrier strips 200, each containing a different medicament dose to be combined, the fluid passageways 50a, 50b from each compartment 202 in each carrier strip 200 are preferably combined in the chamber 60, as illustrated in Figures 9A-9E. This ensures that the user inhales the combination of medicament doses rather than inhaling each dose separately.

In another embodiment (not shown), the separate medicament doses may be kept separate within the device, such that they are combined after exiting the device, for example in the mouth during inhalation by a user of the device. This may provide more control over each medicament dose within the device, for example in relation to mixing each medicament dose with bypass air. In such an arrangement, the chamber 60 may be divided into two, for example the respective air flow paths 50a, 50b may extend separately through the chamber 60, rather than connecting with the chamber 60, such the respective medicament doses do not meet in the chamber 60. As such, the separate medicament doses will instead become mixed during inhalation in the mouth of a user, after they have exited the device.

Thus, in a medicament delivery device in which two different formulations (i.e. carried in separate medicament carriers) are to be mixed, rather than having a mixing chamber 60 that is fluidly connected between a mouthpiece 16 for inhalation, and both release mechanisms 100, as shown in Figure 9A, separate mixing chambers may be provided by air flow paths 50a, 50b being kept separate (e.g. forming separate "mixing chambers") to keep the formulations released from the respective release mechanisms 100 separate prior to inhalation. The separate air flow paths 50a, 50b may extend up to, or even through the mouthpiece 16.

Figures 10A and 10B show a vent 18 for allowing external air to be drawn into the medicament device 2 is located in the side of the housing 4 of the medicament device 2. The vent is, preferably, positioned towards an upper portion of the housing 4. Locating the vent on a side of the housing 4 means that there is no "right way up" for the medicament delivery device 2 to be orientated. As such, this prevents dry powder of the medicament dose escaping through the vents if the device 2 is held upside down, which in this case is in an orientation that is rotated 180° in the vertical plane from the orientation currently shown in Figures 10A and 10B.

In order to ensure that there is sufficient air flow through the device when the device is being used, it is important to make sure that the user cannot cover or block the vent 18 with their fingers when they are holding the device 2 during inhalation. The vent 18 is therefore protected by the cover 20 both when the cover 20 is covering the mouthpiece 16, as shown in Figure 10A, and when the cover 20 is not covering the mouthpiece 16, as shown in Figure 10B. This means that it is not possible for the user's fingers to come into contact with the vent 18 at any point during operation of the device 2 and so it is not possible for the user to block the vent 18. The vent 18 is therefore protected at all times.

When the cover 20 is in the closed position, illustrated in Figure 10A, the cover 20 is arranged to closely cover the vent 18. The seal formed between the vent 18 and the cover 20 is therefore substantially air tight. This prevents the ingress of contaminants, such as fluff and dirt, into the internal mechanism of the delivery device 2. When the cover is in the open position, illustrated in Figure 10B, the cover 20 is arranged to loosely cover the vent 18. The seal formed between the vent 18 and the cover is therefore not air tight and there is no significant resistance to air flow. This ensures that the user is able to drawn air in through the vent 18 and through the housing 4 during inhalation.

The medicament delivery device 2 may include a ratchet mechanism which connects an external lever to be actuated by the user with the internal mechanism that feeds the carrier strip through the release mechanism. In this case, the external lever is the cover 20 and the action of moving the cover 20 from the closed to the open position causes the ratchet mechanism to advance through the indexed mechanism.

The position of a counter window 1006 can also be seen in Figures 10A and 10B. The window 1006 is located in a lower portion of the housing so that it is covered by the cover 20 when the cover is in the open but not covered by the cover 20 when it is in the closed position.

Movement of the cover 20 from the closed to the open position causes the counter to rotate by one count so that the number displayed in the window 1006 changes. Due to the counter decrementing when the cover 20 is opened, the number displayed in the window 1006 is therefore one less that the number of doses actually remaining, because the user has not yet taken that dose. Covering the window 1006 when the cover 20 is open ensures that the user does not get confused as to how many doses are remaining in the delivery device 2.

While the foregoing is directed to exemplary embodiments of the present invention, other and further embodiments of the invention will be apparent to those skilled in the art from consideration of the specification, and may be devised without departing from the basic scope thereof, which is determined by the claims that follow.

## Claims

1. A device for releasing discrete doses of medicament carried in a plurality of spaced-apart compartments (202) on a medicament carrier, each compartment being arranged in a carrier strip (200) and sealed by a sealing layer (206), the device comprising:
a release mechanism (100) arranged to engage between the carrier strip and the sealing layer so as to unseal at least a portion of the compartment and expose the medicament dose contained therein;
**characterised in that** the release mechanism comprises at least one release member (102) arranged to engage between the compartment and sealing layer whereby to remove said at least a portion of the sealing layer; and
wherein the release mechanism comprises two release members.

2. The device of claim 1, wherein the carrier strip passes through the release mechanism when engaged.

3. The device of claim 1 or 2, wherein the release mechanism is arranged to receive the compartment while said at least a portion of compartment is unsealed.

4. The device of any preceding claim, wherein the release mechanism is arranged to remove a portion of the sealing layer from the compartment while leaving a remaining portion of the sealing layer over the compartment.

5. The device of any preceding claim, wherein the release mechanism is arranged to engage and remove at least a portion of the sealing layer from at least one side of the compartment, preferably wherein the release mechanism is arranged to remove portions of the sealing layer from substantially opposed sides of the compartment such that the remaining portion is located between the opposed sides.

6. The device of any preceding claim, further comprising means for engaging with a portion of the sealing layer such that contact is maintained between at least a portion of the sealing layer and the carrier strip when the release mechanism engages the medicament carrier, preferably a substantially central portion of the sealing layer, preferably wherein the means for engaging is a protuberance arranged to contact the sealing layer, preferably to bias or otherwise urge it against the carrier strip.

7. The device of claims 1 to 5, wherein the release mechanism is arranged to separate the sealing layer from the carrier strip such that the sealing layer is completely detached across the full width of the carrier strip over a compartment thereby completely exposing the compartment.

8. The device of any preceding claim, wherein said at least one release member is arranged to engage the medicament carrier such that the carrier strip passes beneath the at least one release member and the sealing layer passes over the at least one release member and/or
wherein the at least one release member has a lower surface arranged to pass closely over each compartment in the carrier strip as the medicament carrier is advanced through the release mechanism, for example so as to substantially seal the portion of compartment where the sealing layer is removed.

9. The device of any preceding claim, wherein the at least one release member has a leading edge arranged to engage between the carrier strip and sealing layer so as to separate the sealing layer from the carrier strip as the carrier moves past the release mechanism, preferably wherein the leading edge has a curved, raised profile along its length.

10. The device of any preceding claim, wherein the at least one release member has a leading edge configured to conform in part to the shape of the perimeter of a compartment and/or wherein the at least one release member has a trailing edge shaped to conform in part to the shape of the perimeter of a compartment.

11. The device of claim 10, wherein at least one of the leading edge and the trailing edge of the at least one release member has a generally curved configuration.

12. The device of any preceding claim, wherein the at least one release member is arranged to extend substantially the entire width of the medicament carrier.

13. The device of any preceding claim, wherein the two release members are arranged in a substantially opposed configuration, such that they are opposed across the medicament carrier, and/or
wherein: either the two release members are spaced apart; or
the two release members are arranged to be coupled together so as to extend substantially the entire width of the medicament carrier.

14. The device of any preceding claim, wherein the at least one projecting member is removable from the release mechanism so as to facilitate engagement with the medicament carrier.

15. The device of any preceding claim, wherein the release mechanism comprises two separable parts that are configured to be coupled together so as to engage with the medicament carrier, and/or
wherein the release mechanism is arranged to remove at least a portion of the sealing layer covering a compartment and to cover said compartment in place of said removed portion of sealing layer, wherein the release mechanism is further arranged to provide a fluid conduit configured to pass air into or through the compartment so as to aerosolise the medicament contained therein.

## Patentansprüche

1. Vorrichtung zum Freigeben einzelner Medikamentendosen, die in einer Vielzahl von beabstandeten Fächern (202) auf einem Medikamententräger getragen werden, wobei jedes Fach in einem Trägerstreifen (200) eingerichtet und durch eine Versiegelungsschicht (206) versiegelt ist, wobei die Vorrichtung umfasst:
einen Freigabemechanismus (100), der dazu eingerichtet ist, zwischen dem Trägerstreifen und der Versiegelungsschicht einzugreifen, um mindestens einen Abschnitt des Fachs zu entsiegeln und die darin enthaltene Medikamentendosis freizulegen;
**dadurch gekennzeichnet, dass** der Freigabemechanismus mindestens ein Freigabeelement (102) umfasst, das dazu eingerichtet ist, zwischen dem Fach und der Versiegelungsschicht einzugreifen, um dadurch den mindestens einen Abschnitt der Versiegelungsschicht zu entfernen; und
wobei der Freigabemechanismus zwei Freigabeelemente umfasst.

2. Vorrichtung nach Anspruch 1, wobei der Trägerstreifen durch den Freigabemechanismus verläuft, wenn er in Eingriff gebracht wird.

3. Vorrichtung nach Anspruch 1 oder 2, wobei der Freigabemechanismus dazu eingerichtet ist, das Fach aufzunehmen, während der mindestens eine Fachabschnitt entsiegelt wird.

4. Vorrichtung nach einem vorstehenden Anspruch, wobei der Freigabemechanismus dazu eingerichtet ist, einen Abschnitt der Versiegelungsschicht von dem Fach zu entfernen, während ein verbleibender Abschnitt der Versiegelungsschicht über dem Fach belassen wird.

5. Vorrichtung nach einem vorstehenden Anspruch, wobei der Freigabemechanismus dazu eingerichtet ist, von mindestens einer Seite des Fachs in mindestens einen Abschnitt der Versiegelungsschicht einzugreifen und diesen zu entfernen, wobei der Freigabemechanismus bevorzugt dazu eingerichtet ist, Abschnitte der Versiegelungsschicht von im Wesentlichen entgegengesetzten Seiten des Fachs derart zu entfernen, dass sich der verbleibende Abschnitt zwischen den entgegengesetzten Seiten befindet.

6. Vorrichtung nach einem vorstehenden Anspruch, die weiter Mittel zum Eingreifen in einen Abschnitt der Versiegelungsschicht derart umfasst, dass Berührung zwischen mindestens einem Abschnitt der Versiegelungsschicht und dem Trägerstreifen aufrechterhalten wird, wenn der Freigabemechanismus in den Medikamententräger, bevorzugt einem im Wesentlichen zentralen Abschnitt der Versiegelungsschicht, eingreift, wobei das Mittel zum Eingreifen bevorzugt ein Vorsprung ist, der dazu eingerichtet ist, die Versiegelungsschicht zu berühren, bevorzugt um sie vorzuspannen oder anderweitig gegen den Trägerstreifen zu drücken.

7. Vorrichtung nach den Ansprüchen 1 bis 5,
wobei der Freigabemechanismus dazu eingerichtet ist, die Versiegelungsschicht derart von dem Trägerstreifen zu trennen, dass die Versiegelungsschicht über einem Fach über die gesamte Breite des Trägerstreifens vollständig abgelöst wird, wodurch das Fach vollständig freigelegt wird.

8. Vorrichtung nach einem vorstehenden Anspruch, wobei das mindestens eine Freigabeelement dazu eingerichtet ist, derart in den Medikamententräger einzugreifen, dass der Trägerstreifen unter dem mindestens einen Freigabeelement verläuft und die Versiegelungsschicht über dem mindestens einen Freigabeelement verläuft, und/oder
wobei das mindestens eine Freigabeelement eine untere Fläche aufweist, die dazu eingerichtet ist, dicht über jedem Fach in dem Trägerstreifen zu verlaufen, wenn der Medikamententräger durch den Freigabemechanismus vorgeschoben wird, um zum Beispiel den Abschnitt des Fachs, an dem die Versiegelungsschicht entfernt wird, im Wesentlichen zu versiegeln.

9. Vorrichtung nach einem vorstehenden Anspruch, wobei das mindestens eine Freigabeelement eine Vorderkante aufweist, die dazu eingerichtet ist, zwischen dem Trägerstreifen und der Versiegelungsschicht einzugreifen, um die Versiegelungsschicht von dem Trägerstreifen zu trennen, wenn sich der Träger an dem Freigabemechanismus vorbei bewegt, wobei bevorzugt die Vorderkante entlang ihrer Länge ein gekrümmtes, erhabenes Profil aufweist.

10. Vorrichtung nach einem vorstehenden Anspruch, wobei das mindestens eine Freigabeelement eine Vorderkante aufweist, die dazu konfiguriert ist, sich zum Teil der Form des Umfangs eines Fachs anzupassen, und/oder wobei das mindestens eine Freigabeelement eine Hinterkante aufweist, die dazu geformt ist, sich zum Teil der Form des Umfangs eines Fachs anzupassen.

11. Vorrichtung nach Anspruch 10, wobei mindestens eine der Vorderkante und der Hinterkante des mindestens einen Freigabeelements eine allgemein gekrümmte Konfiguration aufweist.

12. Vorrichtung nach einem vorstehenden Anspruch, wobei das mindestens eine Freigabeelement dazu eingerichtet ist, sich im Wesentlichen über die gesamte Breite des Medikamententrägers zu erstrecken.

13. Vorrichtung nach einem vorstehenden Anspruch, wobei die zwei Freigabeelemente in einer im Wesentlichen entgegengesetzten Konfiguration derart eingerichtet sind, dass sie einander über den Medikamententräger hinweg entgegengesetzt sind, und/oder
wobei: entweder die zwei Freigabeelemente beabstandet sind; oder
die zwei Freigabeelemente dazu eingerichtet sind, miteinander gekoppelt zu werden, um sich im Wesentlichen über die gesamte Breite des Medikamententrägers zu erstrecken.

14. Vorrichtung nach einem vorstehenden Anspruch, wobei das mindestens eine vorstehende Element von dem Freigabemechanismus entfernt werden kann, um das Eingreifen in den Medikamententräger zu erleichtern.

15. Vorrichtung nach einem vorstehenden Anspruch, wobei der Freigabemechanismus zwei trennbare Teile umfasst, die dazu konfiguriert sind, miteinander gekoppelt zu werden, um in den Medikamententräger einzugreifen, und/oder
wobei der Freigabemechanismus dazu eingerichtet ist, mindestens einen Abschnitt der Versiegelungsschicht, die ein Fach bedeckt, zu entfernen und das Fach an Stelle des entfernten Abschnitts der Versiegelungsschicht zu bedecken, wobei der Freigabemechanismus weiter dazu eingerichtet ist, eine Fluidleitung bereitzustellen, die dazu konfiguriert ist, Luft in oder durch das Fach zu leiten, um das darin enthaltene Medikament zu aerolisieren.

## Revendications

1. Dispositif pour libérer des doses individuelles de médicament transportées dans une pluralité de compartiments espacés (202) sur un support de médicament, chaque compartiment étant agencé dans une bande de support (200) et scellé par une couche d'étanchéité (206), le dispositif comprenant :
un mécanisme de libération (100) agencé pour venir en prise entre la bande de support et la couche d'étanchéité de manière à ouvrir au moins une partie du compartiment et révéler la dose de médicament contenue dans celui-ci ;
**caractérisé en ce que** le mécanisme de libération comprend au moins un élément de libération (102) agencé pour venir en prise entre le compartiment et la couche d'étanchéité de manière à retirer ladite au moins une partie de la couche d'étanchéité ; et
dans lequel le mécanisme de libération comprend deux éléments de libération.

2. Dispositif selon la revendication 1, dans lequel la bande de support passe à travers le mécanisme de libération lorsqu'elle est engagée.

3. Dispositif selon la revendication 1 ou 2, dans lequel le mécanisme de libération est agencé pour recevoir le compartiment en même temps que ladite au moins une partie de compartiment est ouverte.

4. Dispositif selon une quelconque revendication précédente, dans lequel le mécanisme de libération est agencé pour retirer une partie de la couche d'étanchéité du compartiment tout en laissant une partie restante de la couche d'étanchéité sur le compartiment.

5. Dispositif selon une quelconque revendication précédente, dans lequel le mécanisme de libération est agencé pour venir en prise avec et retirer au moins une partie de la couche d'étanchéité sur au moins un côté du compartiment, de préférence dans lequel le mécanisme de libération est agencé pour retirer des parties de la couche d'étanchéité sur des côtés sensiblement opposés du compartiment de telle sorte que la partie restante soit située entre les côtés opposés.

6. Dispositif selon une quelconque revendication précédente, comprenant en outre un moyen pour venir en prise avec une partie de la couche d'étanchéité de telle sorte que le contact soit maintenu entre au moins une partie de la couche d'étanchéité et la bande de support lorsque le mécanisme de libération entre en prise avec le support de médicament, de préférence une partie sensiblement centrale de la couche d'étanchéité, de préférence dans lequel le moyen de mise en prise est une protubérance agencée pour entrer en contact avec la couche d'étanchéité, de préférence pour la solliciter ou bien la pousser contre la bande de support.

7. Dispositif selon les revendications 1 à 5,
dans lequel le mécanisme de libération est agencé pour séparer la couche d'étanchéité de la bande de support de telle sorte que la couche d'étanchéité soit entièrement détachée sur toute la largeur de la bande de support sur un compartiment, exposant ainsi entièrement le compartiment.

8. Dispositif selon une quelconque revendication précédente, dans lequel ledit au moins un élément de libération est agencé pour venir en prise avec le support de médicament de telle sorte que la bande de support passe sous le au moins un élément de libération et que la couche d'étanchéité passe sur le au moins un élément de libération et/ou
dans lequel le au moins un élément de libération présente une surface inférieure agencée pour passer très près sur chaque compartiment dans la bande de support à mesure que le support de médicament est poussé à travers le mécanisme de libération, par exemple de manière à sceller sensiblement la partie du compartiment où la couche d'étanchéité est retirée.

9. Dispositif selon une quelconque revendication précédente, dans lequel le au moins un élément de libération présente un bord d'attaque agencé pour venir en prise entre la bande de support et la couche d'étanchéité de manière à séparer la couche d'étanchéité de la bande de support lorsque le support passe devant le mécanisme de libération, de préférence dans lequel le bord d'attaque présente un profil incurvé et surélevé sur sa longueur.

10. Dispositif selon une quelconque revendication précédente, dans lequel le au moins un élément de libération présente un bord d'attaque configuré pour s'adapter en partie à la forme du périmètre d'un compartiment et/ou dans lequel le au moins un élément de libération présente un bord de fuite formé pour s'adapter en partie à la forme du périmètre d'un compartiment.

11. Dispositif selon la revendication 10, dans lequel au moins l'un parmi le bord d'attaque et le bord de fuite du au moins un élément de libération présente une configuration généralement incurvée.

12. Dispositif selon une quelconque revendication précédente, dans lequel le au moins un élément de libération est agencé pour s'étendre sensiblement sur toute la largeur du support de médicament.

13. Dispositif selon une quelconque revendication précédente, dans lequel les deux éléments de libération sont agencés dans une configuration sensiblement en vis-à-vis, de telle sorte qu'ils sont en vis-à-vis sur le support de médicament, et/ou
dans lequel : soit les deux éléments de libération sont espacés ; soit
les deux éléments de libération sont agencés pour être couplés ensemble de manière à s'étendre sensiblement sur toute la largeur du support de médicament.

14. Dispositif selon une quelconque revendication précédente, dans lequel le au moins un élément saillant est amovible par rapport au mécanisme de libération de manière à faciliter la mise en prise avec le support de médicament.

15. Dispositif selon une quelconque revendication précédente, dans lequel le mécanisme de libération comprend deux parties séparables qui sont configurées pour être couplées ensemble de manière à venir en prise avec le support de médicament, et/ou
dans lequel le mécanisme de libération est agencé pour retirer au moins une partie de la couche d'étanchéité recouvrant un compartiment et pour recouvrir ledit compartiment à la place de ladite partie retirée de la couche d'étanchéité, dans lequel le mécanisme de libération est en outre agencé pour fournir un conduit de fluide configuré pour laisser passer l'air dans ou à travers le compartiment de manière à pulvériser en aérosol le médicament contenu dans celui-ci.
